(19) Europäisches Patentamt
European Patent Office
Office européen des brevets

(11) **EP 2 529 785 A1**

(12) **EUROPÄISCHE PATENTANMELDUNG**

(43) Veröffentlichungstag:
**05.12.2012 Patentblatt 2012/49**

(51) Int Cl.:
*A61N 1/08* (2006.01)    *A61N 1/362* (2006.01)
*A61B 18/14* (2006.01)    *A61B 18/00* (2006.01)

(21) Anmeldenummer: **12165095.6**

(22) Anmeldetag: **23.04.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **10.05.2011   US 201161484242 P**

(71) Anmelder: **BIOTRONIK SE & Co. KG**
**12359 Berlin (DE)**

(72) Erfinder:
• **Dörr, Thomas**
  **12437 Berlin (DE)**
• **Weiss, Ingo**
  **12435 Berlin (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(54) **Vorrichtung für medizinische Anwendungen und elektromedizinisches Implantat**

(57)    Vorrichtung für medizinische Anwendungen, umfassend ein längliches leitfähiges Element mit einem proximalen Ende und einem distalen Ende, wobei das Letztere durch Energieaufnahme aus einem elektromagnetischen Feld einen Temperaturanstieg erfahren kann, mit einem im länglichen leitfähigen Element angeordneten Trennelement zur galvanischen Trennung des proximalen Endes von distalen Ende.

FIG. 4

**Beschreibung**

Die vorliegende Erfindung betrifft eine Vorrichtung für medizinische Anwendungen, welche ein längliches leitfähiges Element mit einem distalen Ende umfasst. Ein solches längliches leitfähiges Element kann z. B. eine Elektrodenleitung sein.

Solche Vorrichtungen werden unter anderem in einer implantierbaren Defibrillationsanordnung 100 erwendet, wie Sie zum Beispiel aus der US 5,531,766 bekannt und in Fig. 1 illustriert ist. Um das Herz H des Patienten P zu stimulieren, steht ein Defibrillator 110 in elektrischer Verbindung mit einer Leitung 101 welche hier eine spezielle Elektrode in Form einer Schockelektrode 104 trägt, die sich an ihrem distalen Ende befindet und im Herzen des Patienten verlegt wird. Die Defibrillation erfolgt in der Regel monopolar über einen Strompfad zwischen dieser Elektrode 104 und einer (nicht dargestellten) Elektrode, die mehr oder weniger entfernt von dem zu stimulierenden Herzen H liegt. Beispielsweise kann das Gehäuse des Defibrillators 102, das die Einheiten zur Detektion von Herzsignalen und Erzeugung von elektrischen Impulsen enthält, als Gegenelektrode wirken.

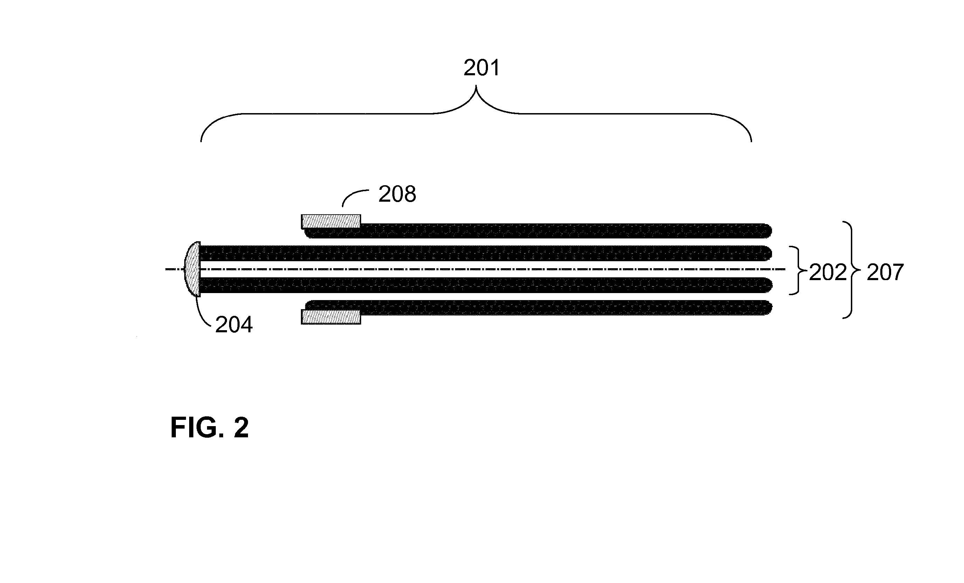

Fig. 2 zeigt das distale Ende einer anderen Leitung 201. Die Spitze der Leitung umfasst zur Ausführung einer sogenannten bipolaren Kardioversion zwei Elektroden 204 und 208. Während die Elektrode 204 einen Kontakt an der Spitze der Leitung 201 bildet, ist die Elektrode 208 als Ringkontakt am Umfang der Leitung ausgestaltet. Die Zuleitung umfasst zwei spiralig gewickelte Leiter 202 und 207, die die Elektroden 204 und 208 elektrisch mit einem (nicht gezeigten) Anschlussstecker am proximalen Ende der Leitung verbinden.

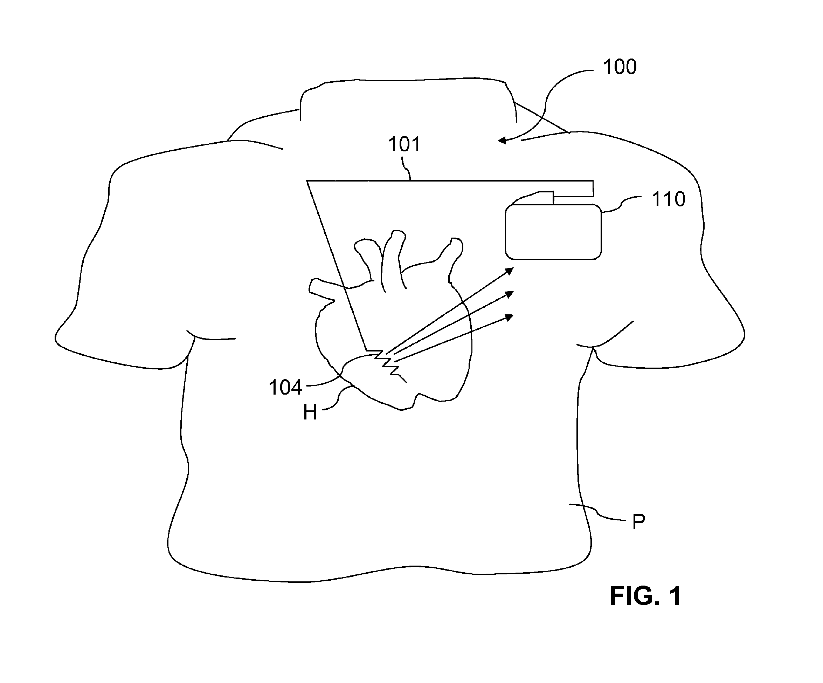

Patienten, die eine konventionelle Defibrillationsanordnung nach Fig. 1 in sich tragen, können derzeit nicht mit Hilfe eines Magnetresonanztomographen (MRT) bzw. MRI-Scanner untersucht werden, da es aufgrund der starken elektromagnetischen Wechselfelder zu einer starken Erwärmung der Leitungsspitze und einer damit zusammenhängenden Schädigung des umliegenden Gewebes kommen kann.

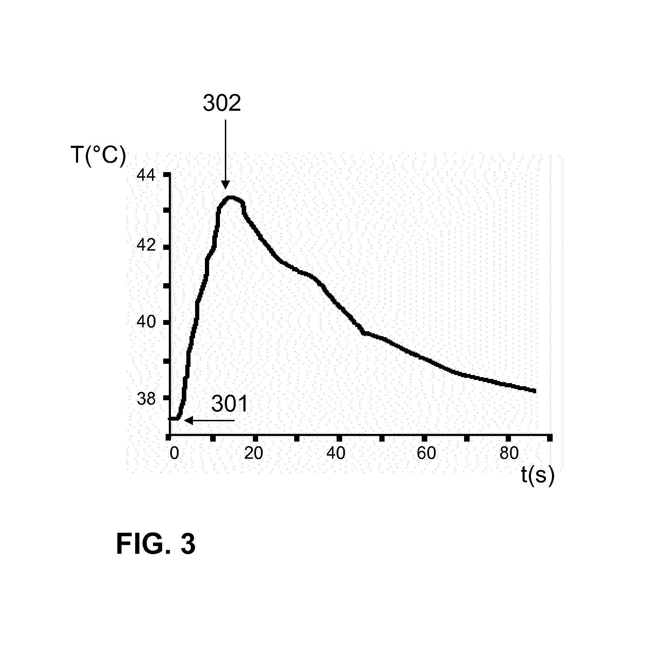

In Fig. 3 ist ein typischer Temperaturverlauf einer Leitungsspitze einer Defibrillationsanordnung in einem Magnetresonanztomographen dargestellt. Mit dem Einschalten des elektromagnetischen Feldes (bezeichnet mit 301) steigt die Temperatur der Leitungsspitze rasch an, wobei die Steilhalt des Anstieges und die maximal auftretende Temperatur stark von der Leitungslage bezogen auf das elektromagnetische Feld des Magnetresonanztomographen abhängig sind. Wird das elektromagnetische Wechselfeld abgeschaltet (bezeichnet mit 302), kühlt sich die Leitungsspitze aufgrund ihrer vergleichsweise geringen Wärmekapazität verhältnismäßig schnell wieder ab.

Zur Lösung des beschriebenen Erhitzungsproblems schlägt die US-Patentanmeldung 2009/0105789 A1 vor, einen temperaturabhängigen Schalter einzusetzen, der bei Erwärmung die Leitungsspitze von der Zuleitung trennt. Ein solcher Schalter hat jedoch eine deutliche Hysterese, der Temperaturbereich ist schlecht einstellbar, und die Leitungsspitze kann nur komplett zu- oder abgeschaltet werden, so dass im abgeschalteten Zustand weder eine Stimulation des Herzens noch eine Ableitung von Signalen möglich ist.

Aus der US 2010/0174348 A1 ist überdies eine implantierbare elektrische Leitung bekannt, die in ihrem Einsatz kompatibel mit einem MRI-Scanner ist. Die Leitung hat hierzu zwei Parallel-Resonatoren, die durch konzentrisch gewendelte isolierte Leitungsdrähte in ihrem Inneren gebildet und auf eine Larmor-Frequenz des Gewebes eines Lebewesens abgestimmt sind, bei dem die Leitung zum Einsatz kommen soll. Als weiterer Mechanismus zur Unterdrückung unerwünschter Einflüsse des im MRI-Scanner bestehenden starken elektromagnetischen Feldes mit HF-Komponente sind im Leitungsverlauf Sperrelemente (cable traps) vorgesehen, die das Auftreten von durch die HF-Komponenten induzierten Strömen auf der Leitung verhindern sollen. Sperrelemente sind bspw. als voneinander beabstandete metallische Hohlzylinder auf dem Außenumfang der Leitung ausgebildet.

Die Erfindung liegt die Aufgabe zugrunde, eine verbesserte Vorrichtung der genannten Art bereit zu stellen, die in starken äußeren Magnetfeldern mit Wechselfeld-Komponente verbesserte Eigenschaften aufweist und einfach aufgebaut und somit kostengünstig herzustellen ist.

Diese Aufgabe wird durch eine Vorrichtung mit den Merkmalen des Anspruchs 1 gelöst. Zweckmäßige Fortbildungen des Erfindungsgedankens sind Gegenstand der abhängigen Ansprüche.

Ein wesentlicher Gedanke der Erfindung besteht darin, den Einfluss starker äußerer Felder durch das Vorsehen einer galvanischen Trennung im Längsverlauf des länglichen leitfähigen Elements der Vorrichtung wesentlich zu reduzieren. Als Trennelement hierfür ist speziell ein Transformator mit einem Kern vorgesehen, welcher derart ausgebildet ist, dass er in einer statischen Magnetfeldkomponente des elektromagnetischen Feldes sättigbar ist. Der Transformator ist kapazitiv derart entkoppelt, dass durch eine Wechselfeldkomponente des elektromagnetischen Feldes im länglichen leitfähigen Element induzierte Hochfrequenzströme über den Transformator wesentlich abgeschwächt werden.

In einer Ausführung der Erfindung ist vorgesehen, dass der sättigbare Kern ein Ferritkern oder ein funktionsgleicher Kern ist, der aus kompakt zusammengefügtem, insbesondere gesintertem, weichmagnetischem Pulver gebildet ist. Als Material dieses Kerns kommen weichmagnetische Pulver auf Co- oder Ni-Basis oder Keramikpulver auf $Mn\text{-}ZnO_x\text{-}$ oder $Ni\text{-}ZnO_x\text{-}$ oder ähnlicher Basis in Betracht, die insbesondere in Kern-Form gepresst und ggf. gesintert sind.

In einer ersten Applikation handelt es sich bei der Vorrichtung um einen Katheter, insbesondere Ablations-, Ballon- oder Einführkatheter oder elektrophysiologischen Katheter. In einer aus derzeitiger Sicht besonders wichtigen Anwendung ist die Vorrichtung ausgebildet als Elektrodenleitung, wobei das distale Ende insbesondere eine Elektrode zur Stimulation eines Herzens, eine ICD-Elektrode, eine transvenöse ICD-Elektrode, eine Elektrode zur Neurostimulation oder eine Elektrode zur tiefen Hirnstimulation aufweist. Hierbei ist der Transformator zur Übertragung von Therapieimpulsen oder -strömen von proximalen zum distalen Ende ausgebildet.

In einer weiteren Ausführung ist der Transformator in den flexiblen Längsverlauf der Elektrodenleitung eingefügt. Alternativ hierzu ist vorgesehen, dass der Transformator in einen Stecker der Elektrodenleitung eingefügt ist. Bei der letzteren Variante ist bei der konstruktiven Ausführung des Transformators weniger Rücksicht auf besonders geringe Abmessungen zu nehmen, jedoch ist die galvanische Trennung hier weit weg vom distalen Leitungsende platziert.

Eine weitere Ausführung der Erfindung sieht vor, dass der Transformator Bestandteil einer in der Elektrodenleitung angeordneten Bandfiltereinrichtung zur selektiven Ausblendung von Hochfrequenzanteilen des elektromagnetischen Feldes ist. Hierbei ist insbesondere ein Ferritkern oder funktionsgleicher Kern des Transformators zur selbsttätigen Anpassung von Filterparametern der Filtereinrichtung an Parameter des elektromagnetischen Feldes ausgebildet.

Vorgeschlagen wird des Weiteren ein elektromedizinisches Implantat mit einer Vorrichtung der oben erläuterten Art sowie einem Impulserzeugergerät (Impulsgenerator, Therapiestrom-Generator), das Mittel zur Ansteuerung der Elektrode(n) der Elektrodenleitung enthält. Im Impulserzeugergerät sind Detektormittel zur Erfassung einer Transformatorimpedanz des in der Elektrodenleitung vorgesehenen Transformators und eingangsseitig mit den Detektormitteln verbundene Anpassungsmittel zur Anpassung des Stimulationsparameters basierend auf der detektierten Transformatorimpedanz vorgesehen.

Für den Einsatz mit einer weiter oben erwähnte Elektrodenleitung, bei der der Transformator Bestandteil einer Bandfiltereinrichtung ist, können auch Detektormittel zur Erfassung von Parametern des äußeren elektromagnetischen Feldes über die sich ergebenden Filterparameter des Filtereinrichtung vorgesehen sein. In einer weiteren Ausgestaltung umfasst die Elektrodenleitung mehrere Kerne mit unterschiedlichen Sättigungsparametern und sind die Detektormittel zur Unterscheidung unterschiedlicher Magnetfeldstärken des elektromagnetischen Feldes aufgrund der Erfassung der Transformatorimpedanz ausgebildet.

Weitere Details der Erfindung und entsprechende Ausführungsformen werden im Folgenden anhand der Figuren beschrieben.

Fig. 1     zeigt einen Grundaufbau einer implantierbaren Defibrillationsanordnung.

Fig. 2     zeigt eine Ausführungsform eines distalen Endes einer Elektrodenleitung.

Fig. 3     illustriert einen Temperaturverlauf einer Spitze einer Leitung während der Einkoppelung eines elektromagnetischen Feldes durch einen Magnetresonanztomographen.

Fig. 4     ist eine schematische Darstellung einer Ausführungsform einer erfindungsgemäßen Vorrichtung.

Fig. 5     ist eine schematische Darstellung einer weiteren Ausführungsform einer erfindungsgemäßen Vorrichtung.

Fig. 6 und 7A-7C     zeigen weitere Ausführungsformen einer erfindungsgemäßen Vorrichtung

Fig. 8     zeigt Sättigungskennlinien verschiedener Kernmaterialien für einen in der erfindungsgemäßen Vorrichtung eingesetzten Transformator.

Fig. 9     zeigt eine weitere Ausführungsform einer erfindungsgemäßen Vorrichtung.

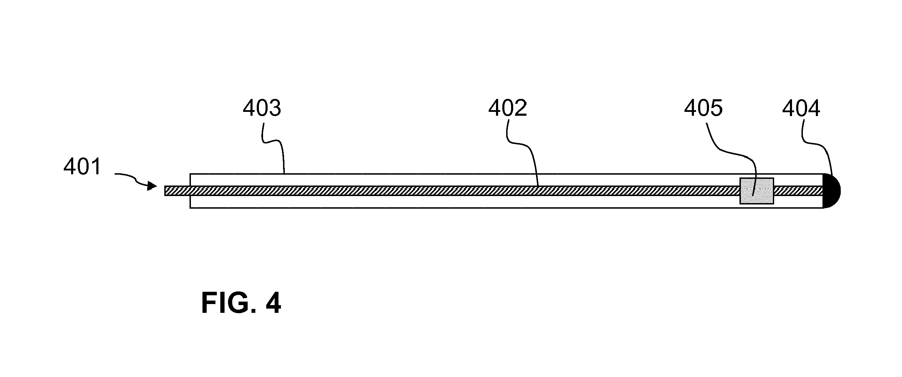

Fig. 4 zeigt eine erfindungsgemäße Vorrichtung 401, die als Elektrodenleitung ausgebildet ist. Ein Leiter (längliches leitfähiges Element) 402 umfasst, eingebettet in eine Isolierhülle 403, eine distale Elektrode (Spitzenelektrode) 404 und ein galvanisches Trennelement 405. Die Wirkung einer induktiven Kopplung eines starken externen Magnetfeldes mit dem Leiter 402 wird durch das galvanische Trennelement 405 im Leiter-Verlauf wesentlich reduziert, womit sich auch eine durch die im Leiter reduzierten Ströme hervorgerufene nachteilige Erwärmung, speziell der Spitzenelektrode 404, erheblich reduzieren lässt. Das Trennelement 405 ist im Übrigen so ausgebildet, dass es für Therapie-Impulse oder -Ströme durchlässig ist, die mit der Elektrodenleitung 401 an Körpergewebe übertragen werden sollen.

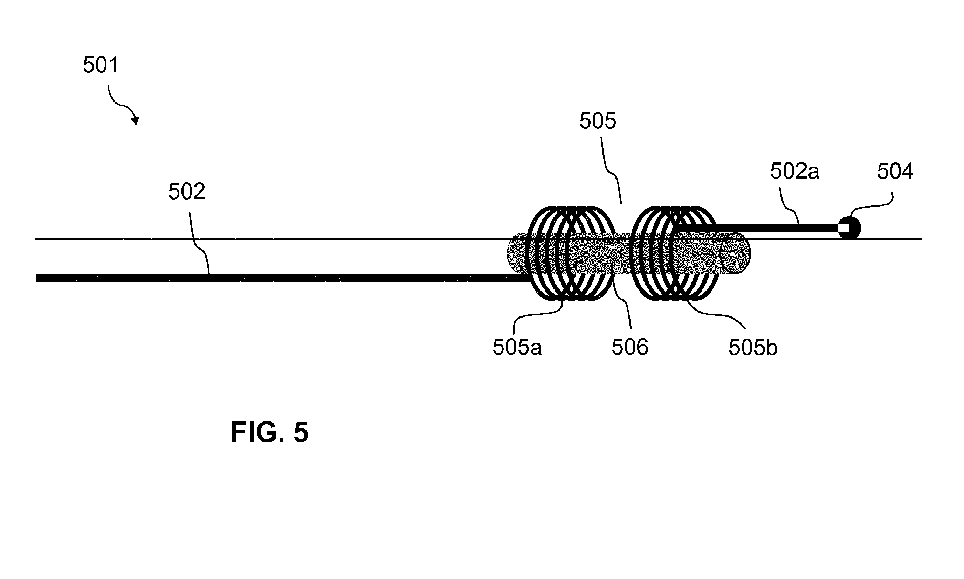

Fig. 5 illustriert als weitere Ausführungsform einer erfindungsgemäßen Vorrichtung 501. Ein erster Elektrodenleiter (längliches leitfähiges Element) 502 umfasst am distalen Ende eine Spitzenelektrode 504. Im Längsverlauf des Elektrodenleiters 502, nahe dem distalen Ende, ist ein Transformator 505 als Trennelement zur Reduzierung von in den Elektrodenleiter eingekoppelten Induktionsströmen vorgesehen. Der Transformator 505 umfasst eine erste, proximal liegende Spulenwicklung 505a und eine zweite, distal liegende Spulenwicklung 505b auf einem gemeinsamen, in einem statischen äußeren Magnetfeld sättigbaren Kerns 506. Die distale Spulenwicklung 505b ist über ein Leiterstück 502a mit der Spitzenelektrode 503 verbunden.

Der Transformator 505 bewirkt eine galvanische Trennung der Spitzenelektrode von dem größeren Teil der (isolierten) Elektrodenleitung 502, die gegenüber einer Wechselfeld-Komponente eines äußeren elektromagnetischen Feldes als Antenne wirkt. In einem solchen Feld geht der Transformatorkern 506 in Sättigung, und die in der Elektrodenleitung 502 induzierten hoch wirkenden Störungen können nicht über die Spitzenelektrode an das Körpergewebe weitergeleitet werden, vorausgesetzt, die kapazitive Kopplung der beiden Spulenwicklungen 505a und 505b ist hinreichend niedrig gewählt.

Die proximale, also antennenseitig liegende Spulenwicklung 505a hat bevorzugt viele Windungen, damit die Induktivität in diesem (proximalen) Transformatorkreis auch bei gesättigtem Transformatorkern noch relativ groß ist, um die "Antennenströme" zu minimieren. Damit kann auf distaler Seite der Transformator 505 z.B. als Abwärtstrafo realisiert werden, was andererseits erfordert, dass über die Elektrodenleitung 505 relativ hohe Spannungen, dafür aber relativ niedrige Ströme geleitet werden. Daher kann zusätzlich der Elektrodenleiter 502 hochohmig gestaltet werden, was dessen Antenneneigenschaften, als seine Affinität gegenüber Störfeldern, abermals verringert. Diese Realisierung ist besonders von Vorteil für Elektrodenleitungen, die ausschließlich für Stimulierungszwecke genutzt werden und keine Abfühlfunktion haben, also keine Signalübertragung vom distalen Ende zum proximalen Ende erfordern.

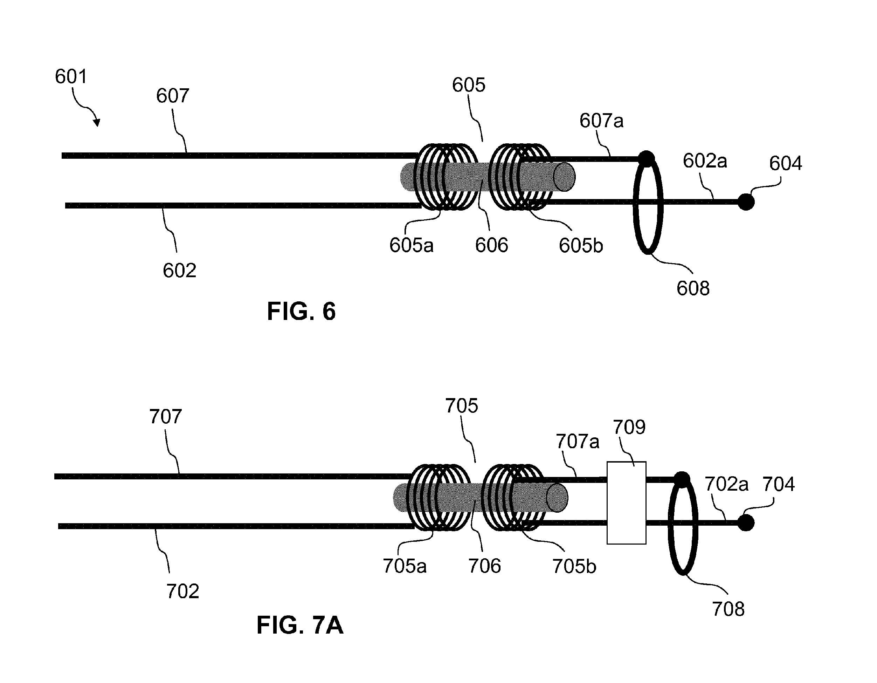

Fig. 6 zeigt eine Abwandlung der in Fig. 5 dargestellten und oben beschriebenen Elektrodenleitung, bei der gleiche Komponenten mit korrespondierenden Bezugsziffern bezeichnet sind. Zur Konfiguration gemäß Fig. 5 kommt ein zweiter Elektrodenleiter 607 mit entsprechendem distalem Leiterabschnitt 607a und einer daran angebrachten Ringelektrode 608 hinzu. Auch dieser gegenüber wirkt der gemeinsame Transformator 605 endkoppelnd und verringert somit die Gefahr einer Erhitzung oder sogar lokalen Verbrennung von Körpergewebe durch die Elektrode, wie oben erläutert.

Ein besonderer Vorteil dieser Realisierung mit Transformator ist insbesondere auch, dass das Risiko ungewünschter Stimulation durch die Gradientenfelder des MRT effektiv vermieden wird.

In einer bevorzugten Ausführung ist die Induktivität L der Spule auf die Gewebeimpedanz (als ohmscher Widerstand R zu betrachten) zwischen den Elektroden 604 und 608 so abgestimmt, dass eine gewünschte Pulsbreite des Stimulationspulses erzielt wird, z.B. dass also die Zeitkonstante tau=L/R diese gewünschte Dauer hat.

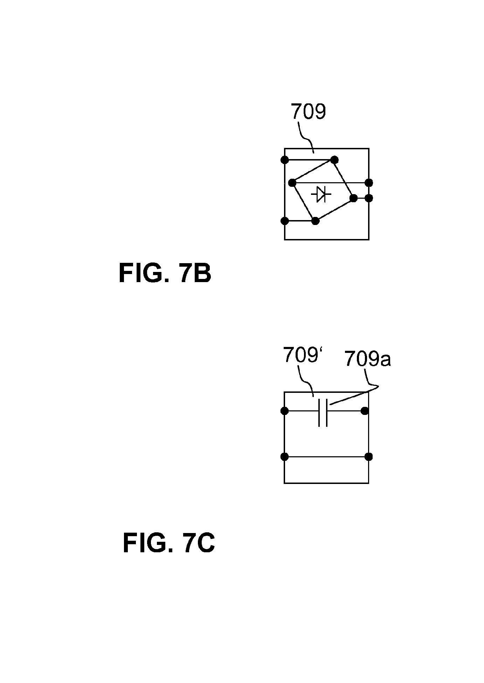

Die Figuren 7A bis 7C zeigen eine weitere Abwandlung der in Fig. 6 gezeigten Elektrodenleitung, und zwar eine durch Hinzufügung eines Signalwandlers 709 geschaffene. Der Signalwandler 709 dient dazu, das zu einem therapeutischen Zweck über die Elektrodenleitung 701 übertragene Wechselspannungssignal bzw. einen Impuls in eine für die Stimulation günstigere Signalform zu wandeln. Wie in Fig. 7B skizziert, kann hierzu eine Gleichrichterschaltung dienen. Wie in Fig. 7C skizziert, kann ein modifizierter Signalwandler 709' einen Kondensator 709a umfassen, dessen Kapazität C so auf die Induktivität der distalen Spulenwicklung 705b abgestimmt ist, dass der ohmsche Widerstand des Leiterabschnitts 702a, die Spuleninduktivität und die Kondensatorkapazität einen Schwingkreis mit kritische Dämpfung realisieren, d.h. $R = \sqrt{L/C}$ realisiert ist.

In einer alternativen Ausprägung der Erfindung wird die Tatsache ausgenutzt, dass ein magnetischer Spulenkern bei einer bestimmten, definierten Feldstärke sättigt und sich dabei die Induktivität der Spule verringert. Weiterhin wird ausgenutzt, dass die HF-Frequenz f des MRT über die Larmorbeziehung direkt proportional an die statische Feldstärke B0 des MRT gebunden ist.

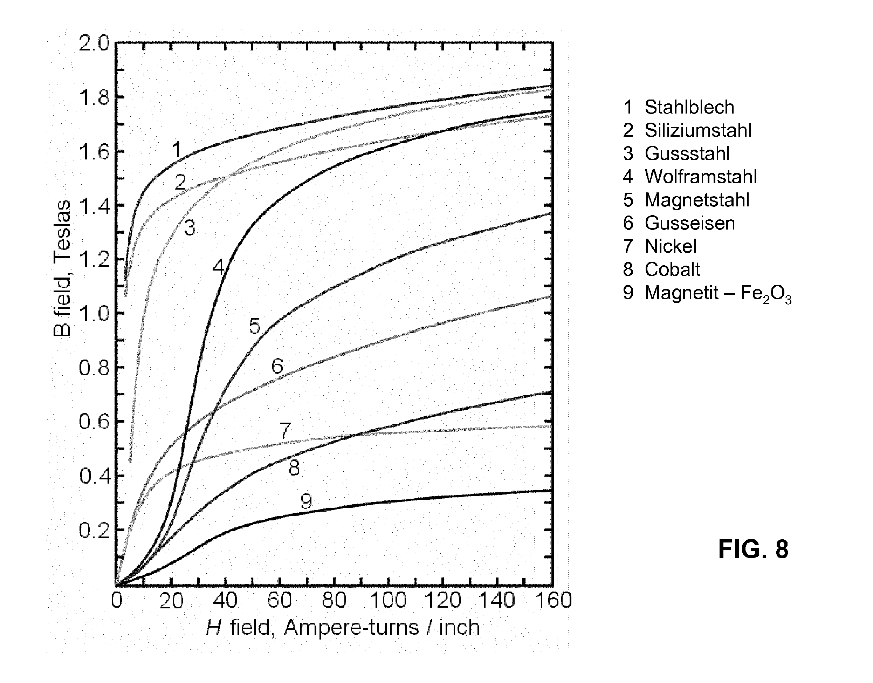

In Fig. 8 ist das Sättigungsverhalten verschiedener Kernmateralien dargestellt.

Folgende Anwendungsfälle können genutzt werden:

Anwendungsfall 1: Bildung einer Längsinduktivität zur Realisierung einer hohen Impedanz vor dem jeweiligen Elektrodenpol, unter Auswahl des Materials des Transformatorkerns derart, dass es in einem statischen Magnetfeld nicht sättigt und dort der Kern wirksam und die Spulenimpedanz groß ist, während in hohem statischen Magnetfeld der Kern sättigt und die Induktivität der Spule klein ist. Wirkt in diesem Fall aber eine hochfrequente Feldkomponente, so wird durch diese die relativ geringe Induktivität dahingehend "kompensiert", dass die resultierende Spulenimpedanz gleichwohl relativ hoch wird.

Anwendungsfall 2: Alle Realisierungen von MRI-kompatiblen Elektroden/Kathetern, in denen LC-Schwingkreise vorhanden sind. Sättigt hier der Kern in einem externen Magnetfeld mit hoher statischer Feldstärke, wird die Induktivität der Spulenwicklung klein und folglich die Resonanzfrequenz hoch. Durch die Geometrie der Spule und des Kerns, insbesondere Bestimmung des Teils des Spulenquerschnitts, der vom Kern ausgeführt wird, kann die Kennlinie, mit der sich die Induktivität und damit die Frequenz verändern, anwendungsgerecht eingestellt werden.

So kann dieselbe Elektrode für MRT-Magnetfeldstärken von 1.5 T und 3 T kompatibel gemacht werden, indem ein Kernmaterial gewählt wird, das zwischen 1.5 T und 3T sättigt (siehe Fig. 8). Des Weiteren können mehrere Spulen mit verschiedenen Kernen oder verschiedene Kerne in einer Spule dazu genutzt werden, dieselbe für z.B. 1T, 1.5 T und 3T kompatibel zu machen. Die Kernmaterialien sättigen zwischen 1 und 1,5 T bzw. 1,5 und 3T.

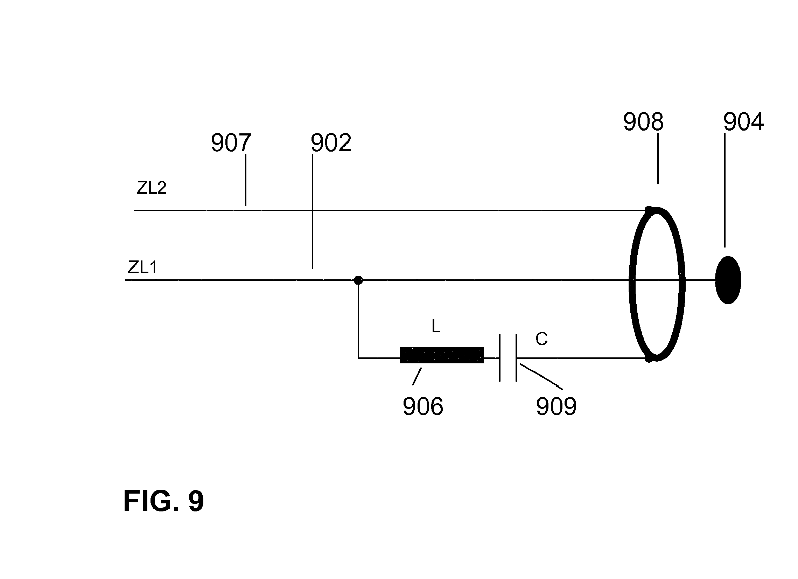

In Fig. 9 (in der Bezugsziffern in Anlehnung an die Bezeichnung der Komponenten in Fig. 6 bis 7C vergeben wurden) ist in einem Ersatzschaltbild die Ableitung der MRT-induzierten Ströme auf eine Ringelektrode 908 dargestellt. Das Prinzip ist es, mit dem gezeigten Schwingkreis im Resonanzfall Spitze 904 und Ring 908 kurzzuschließen. 908 kann dabei ein therapeutisch genutzter Ring sein (Wahrnehmung von elektrischen Signalen und/oder Abgabe von elektrischen Signalen) oder ein erdfreier Ring (floating ring) sein. Die Vorteile gegenüber anderen bekannten Lösungen sind die kleinen Bauelementegrößen, insbesondere Spulen mit sehr kleiner Induktivität, die damit gut mit dem Elektrodendesign vereinbar sind.

Die Resonanzfrequenz berechnet sich nach:

$$f_0 = \frac{1}{2\pi\sqrt{LC}}$$

Für einen 1,5T MRT ist somit bei einer Kapazität C = 1pF "nur" eine Induktivität von etwa 6,5μH im Resonanzkreis nötig. Eine solche Anordnung ist ggf. hinter einer Ringelektrode unterzubringen. Um diese Anordnung auch in einem 3T-MRT verwenden zu können, ist die Spule L 906 im Resonanzkreis mit einem Kern ausgestattet, der ein Sättigungsverhalten oberhalb von 1,5T aber unterhalb von 3T aufweist und so dimensioniert ist, dass die Induktivität im Resonanzkreis bei 3T (gesättigter Kern) auf etwa 1,5μH absinkt und damit die Resonanzfrequenz für ein 3T-MRT eingestellt wird. Eine LC-Reihenschaltung wird bevorzugt, da die Impedanz bei der Resonanzfrequenz geringer ist als bei der Verwendung allein eines Kondensators C, obwohl die Realisierung mit einem nur einen Kondensator (also ohne Spule) zum Verbinden eines Leiters 902 der Elektrodenleitung mit einer Ringelektrode 908 (oder einem anderen Pol mit einer anderen Form, wie Beispielsweise eine ICD-Schockwendel oder Schockelektrode) auch Teil der Erfindung ist.

Auch kann anstelle der Spule L 906 ein Transformator in der vorstehenden Konfiguration verwendet werden.

Ähnliche bzw. funktionsgleiche Anordnungen wie die oben beschriebene können auch mit alternativen Einrichtungen zur Filterung der MRT-induzierten HF-Ströme angewendet werden.

Geeignete Kernmaterialien, bei denen zwischen 1,5T und 3T eine Sättigung einsetzt, sind vor allem aus Cobalt-Eisen mit einem Anteil von 47-50% Cobalt. Ebenso ist Silizium-Eisen mit 3-4% Silizium geeignet.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

**Patentansprüche**

1. Vorrichtung für medizinische Anwendungen, umfassend ein längliches leitfähiges Element mit einem proximalen Ende und einem distalen Ende, wobei das Letztere durch Energieaufnahme aus einem elektromagnetischen Feld einen Temperaturanstieg erfahren kann, mit einem im länglichen leitfähigen Element angeordneten Trennelement zur galvanischen Trennung des proximalen Endes vom distalen Ende.

2. Vorrichtung nach Anspruch 1, wobei das Trennelement ein Transformator mit einem Transformatorkern ist, welcher in einer statischen Magnetfeldkomponente des elektromagnetischen Feldes sättigbar ist, und der Transformator kapazitiv derart entkoppelt ist, dass durch eine Wechselfeldkomponente des elektromagnetischen Feldes im länglichen leitfähigen Element induzierte Hochfrequenzströme über den Transformator wesentlich abgeschwächt werden.

3. Vorrichtung nach Anspruch 2, wobei der sättigbare Kern ein Ferritkern oder ein funktionsgleicher Kern ist, der aus kompakt zusammengefügtem, insbesondere gesintertem, weichmagnetischem Pulver gebildet ist.

4. Vorrichtung nach Anspruch 2 oder 3, wobei mehrere sättigbare Kerne mit unterschiedlichen Sättigungsparametern, insbesondere unterschiedlich steiler Sättigungskennlinie, vorgesehen sind.

5. Vorrichtung nach einem der vorangehenden Ansprüche, ausgebildet als Katheter, insbesondere Ablations-, Ballon- oder Einführkatheter oder elektrophysiologischer Katheter.

6. Vorrichtung nach einem der Ansprüche 1 bis 4, ausgebildet als Elektrodenleitung, wobei das distale Ende insbesondere eine Elektrode zur Stimulation eines Herzens, eine ICD-Elektrode, eine transvenöse ICD-Elektrode, eine Elektrode zur Neurostimulation oder eine Elektrode zur tiefen Hirnstimulation aufweist und wobei der Transformator zur Übertragung von Therapieimpulsen oder -strömen von proximalen zum distalen Ende ausgebildet ist.

7. Vorrichtung nach Anspruch 6, wobei der Transformator in den flexiblen Längsverlauf der Elektrodenleitung eingefügt ist.

8. Vorrichtung nach Anspruch 6, wobei der Transformator in einen Stecker der Elektrodenleitung eingefügt ist.

9. Vorrichtung nach einem der Ansprüche 6 bis 8, wobei der Transformator Bestandteil einer in der Elektrodenleitung angeordneten Bandfiltereinrichtung zur selektiven Ausblendung von Hochfrequenzanteilen des elektromagnetischen Feldes ist.

10. Vorrichtung nach Anspruch 9, wobei ein Ferritkern oder funktionsgleicher Kern des Transformators zur selbsttätigen Anpassung von Filterparametern der Filtereinrichtung an Parameter des elektromagnetischen Feldes ausgebildet ist.

11. Elektromedizinisches Implantat mit einer Vorrichtung nach einem der Ansprüche 6 bis 10 und einem Impulserzeugergerät, welches Mittel zur Ansteuerung der oder einer Elektrode der Elektrodenleitung basierend auf einem Stimulationsparameter aufweist, wobei im Impulserzeugergerät Detektormittel zur Erfassung einer Transformatorimpedanz des in der Elektrodenleitung vorgesehenen Transformators und eingangsseitig mit den Detektormitteln verbundene Anpassungsmittel zur Anpassung des Stimulationsparameters basierend auf der detektierten Transformatorimpedanz vorgesehen sind.

12. Implantat nach Anspruch 11, wobei in der Elektrodenleitung mehrere Kerne mit unterschiedlichen Sättigungsparametern vorgesehen und die Detektormittel zur Unterscheidung unterschiedlicher Magnetfeldstärken des elektromagnetischen Feldes aufgrund der Erfassung der Transformatorimpedanz ausgebildet sind.

**FIG. 1**

FIG. 2

EP 2 529 785 A1

FIG. 3

EP 2 529 785 A1

FIG. 4

**FIG. 5**

EP 2 529 785 A1

FIG. 6

FIG. 7A

709

**FIG. 7B**

709'  709a

**FIG. 7C**

EP 2 529 785 A1

1 Stahlblech
2 Siliziumstahl
3 Gussstahl
4 Wolframstahl
5 Magnetstahl
6 Gusseisen
7 Nickel
8 Cobalt
9 Magnetit – $Fe_2O_3$

FIG. 8

EP 2 529 785 A1

EP 2 529 785 A1

907    902

908    904

ZL2

ZL1

L        C

906      909

**FIG. 9**

Europäisches
Patentamt

European
Patent Office

Office européen
des brevets

**EUROPÄISCHER RECHERCHENBERICHT**

Nummer der Anmeldung

EP 12 16 5095

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2005/197677 A1 (STEVENSON ROBERT A [US]) 8. September 2005 (2005-09-08) * Absätze [0063], [0064], [0219]; Abbildungen 17,18 * ----- | 1-12 | INV. A61N1/08 ADD. A61N1/362 A61B18/14 A61B18/00 |
| X | US 2007/112398 A1 (STEVENSON ROBERT A [US] ET AL) 17. Mai 2007 (2007-05-17) * Zusammenfassung; Abbildung 20 * ----- | 1 | |

RECHERCHIERTE
SACHGEBIETE (IPC)

A61N

Der vorliegende Recherchenbericht wurde für alle Patentansprüche erstellt

| Recherchenort | Abschlußdatum der Recherche | Prüfer |
|---|---|---|
| München | 29. August 2012 | Edward, Vinod |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

                                                  
& : Mitglied der gleichen Patentfamilie, übereinstimmendes
    Dokument

EPO FORM 1503 03.82 (P04C03)

EP 2 529 785 A1

**ANHANG ZUM EUROPÄISCHEN RECHERCHENBERICHT**
**ÜBER DIE EUROPÄISCHE PATENTANMELDUNG NR.**

EP 12 16 5095

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-08-2012

| Im Recherchenbericht angeführtes Patentdokument | Datum der Veröffentlichung | Mitglied(er) der Patentfamilie | Datum der Veröffentlichung |
|---|---|---|---|
| US 2005197677 A1 | 08-09-2005 | CA 2516034 A1<br>EP 1632265 A1<br>JP 2006068541 A<br>US 2005197677 A1 | 02-03-2006<br>08-03-2006<br>16-03-2006<br>08-09-2005 |
| US 2007112398 A1 | 17-05-2007 | EP 1945297 A2<br>JP 2009514617 A<br>US 2007112398 A1<br>US 2011066212 A1<br>US 2011201912 A1<br>WO 2007102893 A2 | 23-07-2008<br>09-04-2009<br>17-05-2007<br>17-03-2011<br>18-08-2011<br>13-09-2007 |

EPO FORM P0461

Für nähere Einzelheiten zu diesem Anhang : siehe Amtsblatt des Europäischen Patentamts, Nr.12/82

17

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5531766 A
- US 20090105789 A1
- US 20100174348 A1